Europäisches Patentamt

European Patent Office

Office européen des brevets

(11) Publication number: **0 093 454**
A1

# EUROPEAN PATENT APPLICATION

(21) Application number: **83104353.4**

(22) Date of filing: **03.05.83**

(51) Int. Cl.³: **C 07 C 7/13**
**C 07 C 15/08**

---

(30) Priority: **04.05.82 IT 2105982**

(43) Date of publication of application:
**09.11.83 Bulletin 83/45**

(84) Designated Contracting States:
**BE DE FR GB NL**

(71) Applicant: **SISAS Società Italiana Serie Acetica Sintetica S.p.A.**
**Largo Corsia dei Servi 3**
**I-20122 Milano(IT)**

(72) Inventor: **Santacesaria, Elio**
**Via Fratelli Rosselli 20**
**I-20139 Milano(IT)**

(72) Inventor: **Codignola, Franco**
**Largo Corsia dei Servi 3**
**I-20122 Milano(IT)**

(72) Inventor: **Gelosa, Davino**
**Via Golgi 76**
**I-27100 Pavia(IT)**

(72) Inventor: **Carra, Sergio**
**Viale Piceno 44**
**I-20121 Milano(IT)**

(74) Representative: **Struif, Bernward, Dipl.-Chem. Dr. et al,**
**Patentanwaltsbüro Tiedtke, Bühling, Kinne Gruppe, Pellmann, Struif, Dragotti Bavariaring 4**
**D-8000 München 2(DE)**

---

(54) A process for the separation of mixtures of aromatic hydrocarbons into single components.

(57) Process for the separation of aromatic hydrocarbons (e.g. xylenes) from their mixture, in which the mixture is passed in the vapour phase at 152, 5-250°C over a potassium exchanged zeolite Y which has been saturated with iso-propylbenzene - i.e. the desorbent - vapours.

EP 0 093 454 A1

This invention concerns a process for the separation of aromatic hydrocarbon mixtures in the single components.

The ortho, para, metaxylene and ethylbenzene $C_8$ isomers, when at pure state, are used industrially as raw materials for the production of various petrochemicals which, in their turn, are used to prepare plastics, resins and something else.

One knows very well that the separation of the single isomers from their mixtures is a very difficult operation, because of the proximity of the boiling points and because of the similarity of the other chemical-physical characteristics.

More recently, the separation of said isomers was fulfilled through selective adsorption on zeolite-type materials. Such a separation can be theoretically considered as a two-stage process: an adsorption one, where a part of the components of the $C_8$ fraction is fixed to the zeolite material, thus undergoing to a first separation; and a desorption one, where the product fixed to the zeolite is separated by adding another component (desorbent) which has an affinity for the solid comparable to the one of the adsorbed material. Of the two steps, the desorption one is usually slower and affects therefore the rate of the total process.

In the US Patent No. 3,773,846 is disclosed a process for the separation, on zeolite materials, of meta- and paraxylene from a liquid fraction of $C_8$ aromatic compounds. In such a process the used desorbent is indifferently one of the xylene fractionation products, preferably toluene or diethylbenzene; it is important to emphasize that in the process being described in this patent, the products being used as desorbent are reputed fully equivalent and accordingly not important. However, as it will be better stated later on, toluene is not especially suitable to desorb the components being fixed to the zeolite, ethylbenzene cannot in its turn be separated from the other isomers.

Hence the requirement to take advantage of a complicate serial of traditional operations, such as the fractionate crystallization and so on.

In the Italian patent application No. 24044 A/80 is disclosed the use of zeolite material having large pores of Y type exchanged with potassium.

The so treated material adsorbs very well the paraxylene and in the process is used toluene as desorbent.

The desorption by means of this type of aromatic is however rather slow; for the purposes of the above mentioned application that are substantially those to separate a pure metaxylene vapor fraction, toluene is more suitable as it has affinity for the solid that is intermediate between the metaxylene and the ethylbenzene ones.

Paraxylene is however hardly displaced from toluene, that therefore must be used in large quantities during the desorption process, so to make the removal complete.

The object of this invention is to carry out an industrial process that allows to separate the single components from $C_8$ aromatic hydrocarbon mixtures.

This invention solves the problem to supply a process to separate quickly and in a selective way the component being adsorbed on the zeolite material without using excessive quantities of desorbent.

These and other objects can be attained by the process of the invention to separate the aromatic hydrocarbons mixtures in the single components by passing the mixtures to vapor phase on an adsorption bed formed by a potassium-exchanged Y zeolite and being previously saturated by desorbent in the vapor state, said process being substantially characterized by the fact that the desorbent is isopropylbenzene, the zeolite material bed is being kept to a temperature between 152.5 and 250°C included, the zeolite bed being treated during the desorption phase with an isopropylbenzene feed in vapor state which separates the pure components fractions, with the exception for the desorbent presence, and fractions composed of mixtures of two or more of said components in presence of desorbent.

According to another characteristic of this invention, said fractions being desorbed from the zeolite material bed are mostly enriched in the paraxylene component which separates quickly together with the other components, into cuts whose paraxylene molar fraction is predominantly higher if compared to the one of the other components in the mixtures.

The zeolite being used in this invention is under the form of extruded granules in which the sodium ions are fully exchanged with potassium. Futhermore the zeolite material bed is saturated by isopropylbenzene in vapor state.

The operative conditions are as follows:

higher temperature or equal to the boiling temperature of isopropylbenzene at the process pressures, usually not higher than 250°C and preferably equal to 170°C;

pressure not higher than 2 atm, preferably at atmospheric desorbent pressure; feed velocity on zeolite bed between 100 and 350 $h^{-1}$.

The selectivity values as obtainedfor the adsorption of the various xylenes in vapor state on the zeolite being saturated with isopropylbenzene, in comparison with metaxylene and measured at 170°C, are as follows:

TABLE 1

| Component | Selectivity |
|---|---|
| Ortoxylene | 1 |
| Paraxylene | 2.4 |
| Ethylbenzene | 1.6 |

The desorbent is the isopropylbenzene which as, hereinbefore described, is also used to saturate, in vapor phase, the adsorbent bed before the mixture is fed.

The action of the desorbent is to remove the adsorbed components on the zeolite, enriching the top mixture of the component that has a lower affinity for the solid and that emerges from the column at the concentration a little higher than the one that it had during the feed.

The isopropylbenzene being used in this invention allows separation, during the desorption, of all the components of the mixtures in the pure state, more rapidly in comparison to the conventional processes (which require the intervention of recycles or other auxiliary operations) without taking advantage of an excess of desorbent.

Unlike the traditional processes, the xylene-fractions produced according to this invention are enriched more and more in the single components, including paraxylene that is present with high molar fractions in the cuts separated from desorption.

The enrichment of paraxylene in the vapor stream emerging from the column in the desorption phase, which reaches almost unitary molar fractions, would not be possible, if the desorption were made by using toluene or other aromatic derivatives instead of isopropylbenzene.

Example 6, hereinafter described, clearly shows this difference in the separation results; from these results one may observe that, in spite of what expected, the choise of the desorbent affects the type and the quality of the desorbed fractions and one notices especially that the isopropylbenzene is more active as desorbent, if compared to those being used in the traditional processes, being lower either the time necessary to let all the components emerge, either the quantity of the desorbent being necessary so that it can take place.

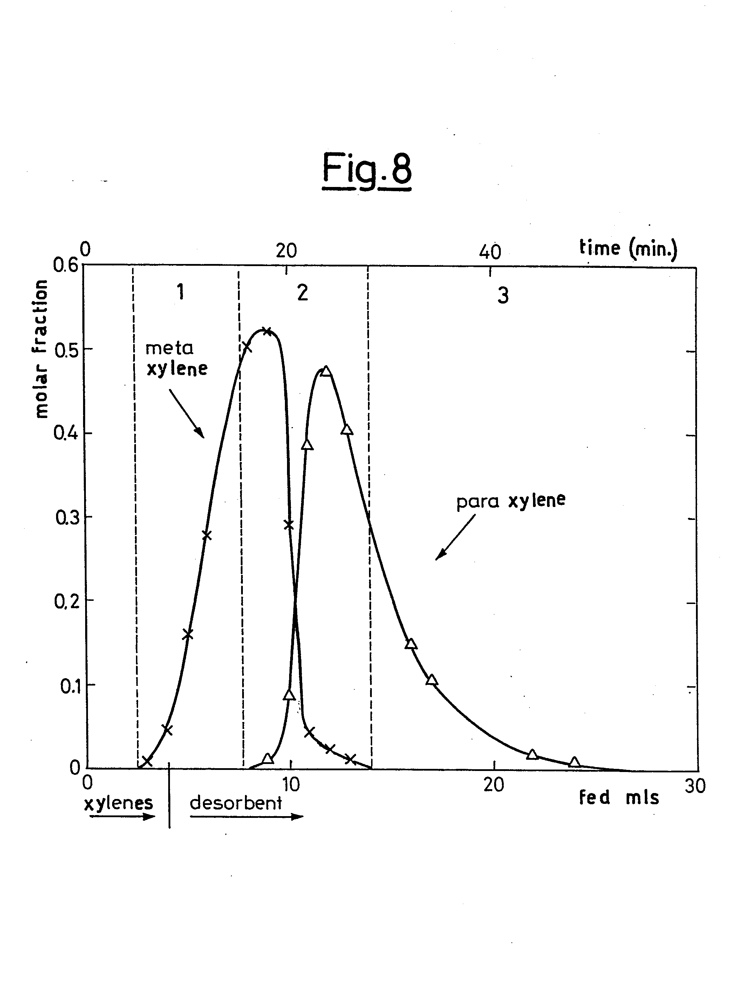

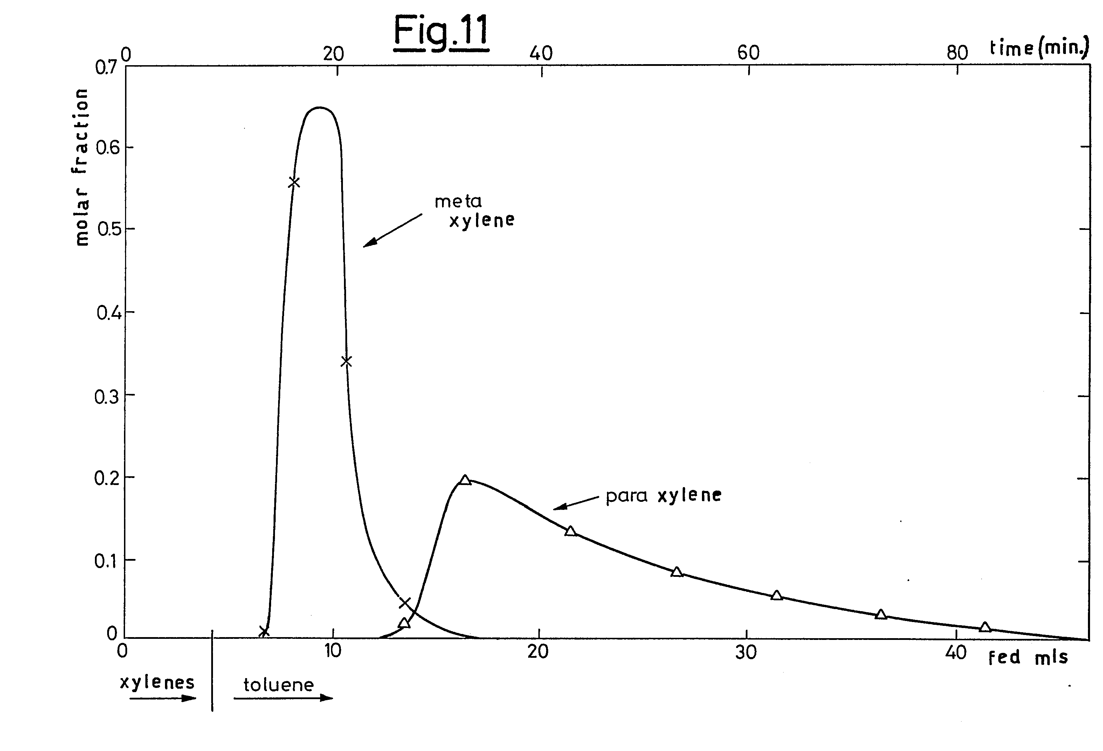

The above effect clearly results from the desorption curves of the enclosed figures, which represent the component molar fraction that is obtained at the outlet of the column depending on the time and on the desorbent quantity as fed, i.e.isopropylbenzene. It is especially of interest the comparison between the desorption curves of figures 8 and 11, both referred to the meta/paraxylene separation respectively carried out with isopropylbenzene and toluene; after checking these figures, it results to be confirmed, among other things, the separation of paraxylene with concentration levels notably higher for the case of isopropylbenzene, and the higher rate by which such a separation takes place, such a rate which obviously implies lower total amounts of the desorbent. Without setting up any undue limitation to this invention, it seems to be plausible to justify this phenomenon with the fact that isopropylbenzene presents a higher affinity against the solid in comparison to the other types of desorbent being used till nowadays.

According to the conditions, it is possible to operate in different manners.

For istance it is possible to feed quantities of xylenes lower than the load capacity of the zeolite, by using the desorbent as a fluid carrier; in this way one may obtain the almost complete separation between meta and paraxylene. This results, among other things, from example 4 where it is also shown the effect of the variations of the linear rate of the desorbent feeding to the separation.

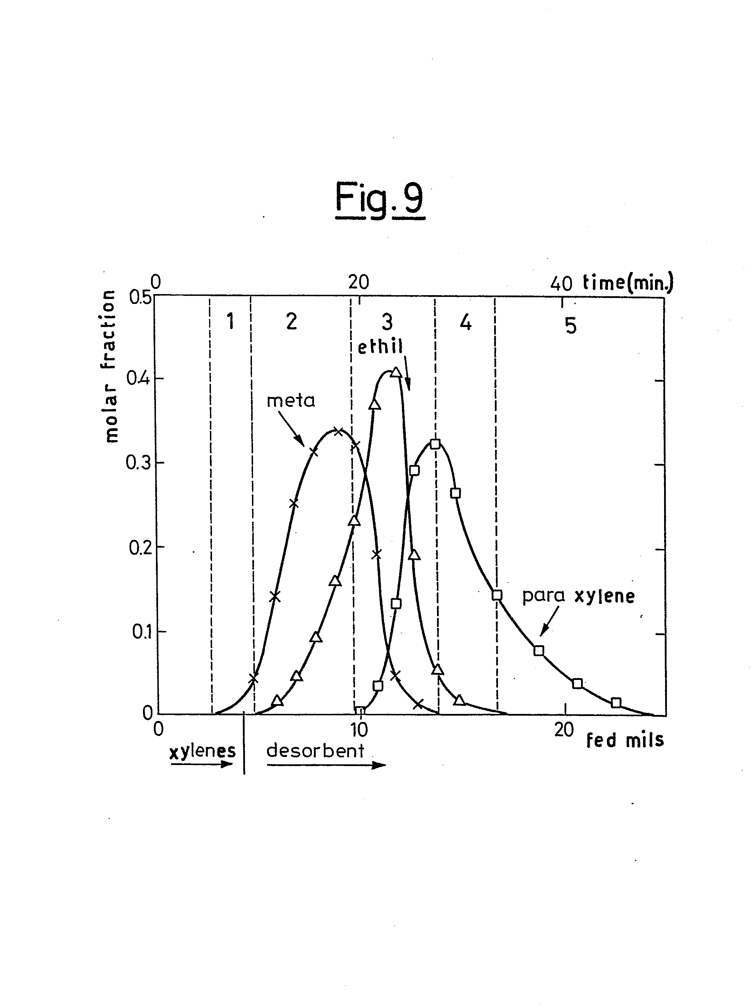

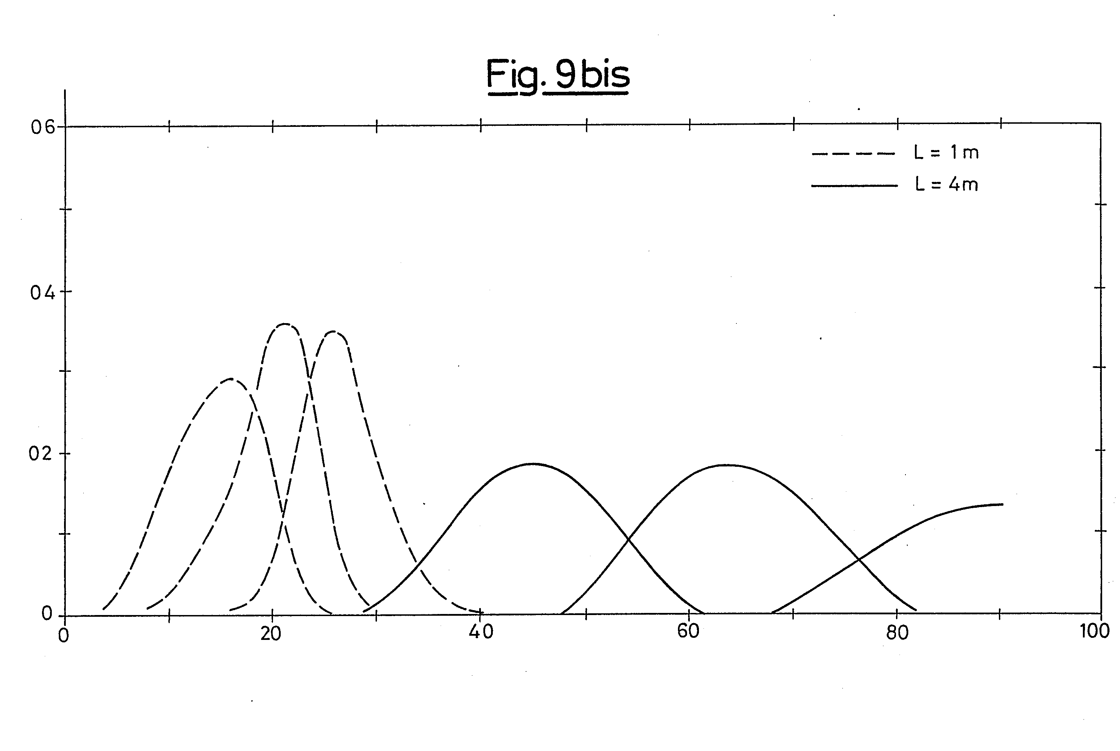

By increasing the lengh of the columns at parity of diameter as shown on the desorption curves on figure 9 bis, one observes the complete separation of the fractions of the pure components, excepted for the desorbent.

In the following examples reference is made to different adsorption and desorption curves, being experimentally obtained by using for the analysis the vapor flowing out from the column and a gas chromatograph mounted on line, equipped with an automatic sampling valve kept at the temperature of the column.

## EXAMPLE 1

Breakthrough curves either in adsorption and in desorption for a binary mixture of metaxylene and paraxylene (1:1 in moles) using isopropylbenzene as desorbent.

The column being used is made of copper, has a diameter of 1 cm and a lenght of 1 m. It contains 54.26 g of potassium - exchanged zeolite being pretreated as described beforehand. In the adsorption test, a mixture containing 50% in moles of the two isomers, i.e. metaxylene and paraxylene, has been fed, after vaporisation, at atmospheric pressure, on the adsorbent bed kept at 170°C, with a load of 0.25 $cm^3$/min. of liquid, at 25°C. The adsorbent bed was previously saturated with isopropylbenzene.

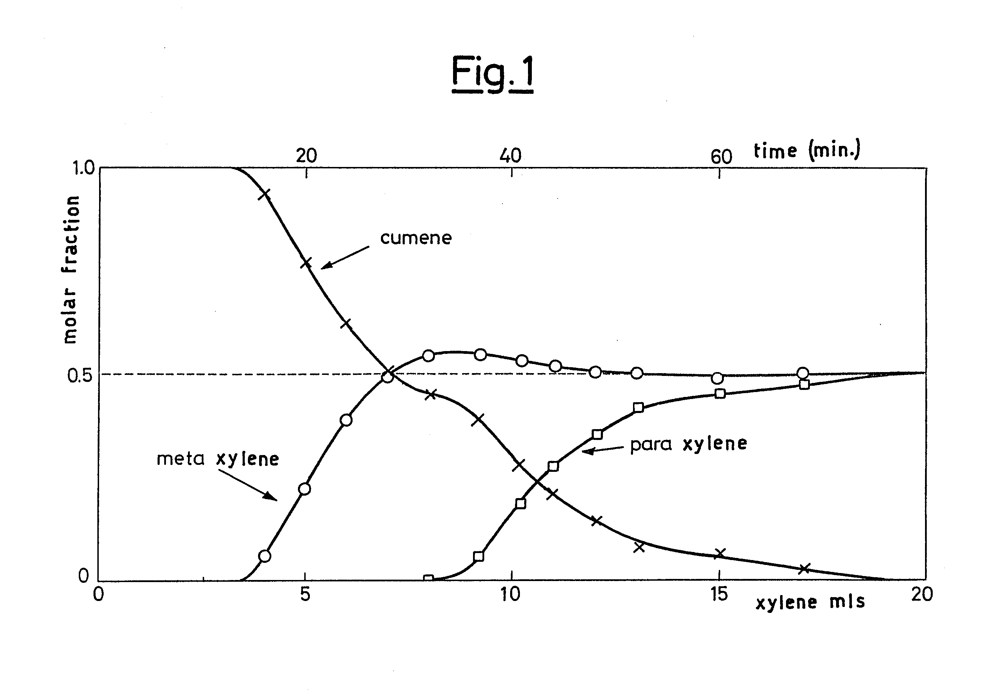

The breakthrough curves of fig. 1 are obtained. From these curves it is possible to realize that the quantities of meta and paraxylene being adsorbed are respectively $3.5 \times 10^{-4}$ moles/g and $8.4 \times 10^{-4}$ moles/g.

The selectivity values are shown in Table 1. The quantity of metaxylene that is fully separated from paraxylene represents about 35% by weight in comparison to that being fed up up to that stage.

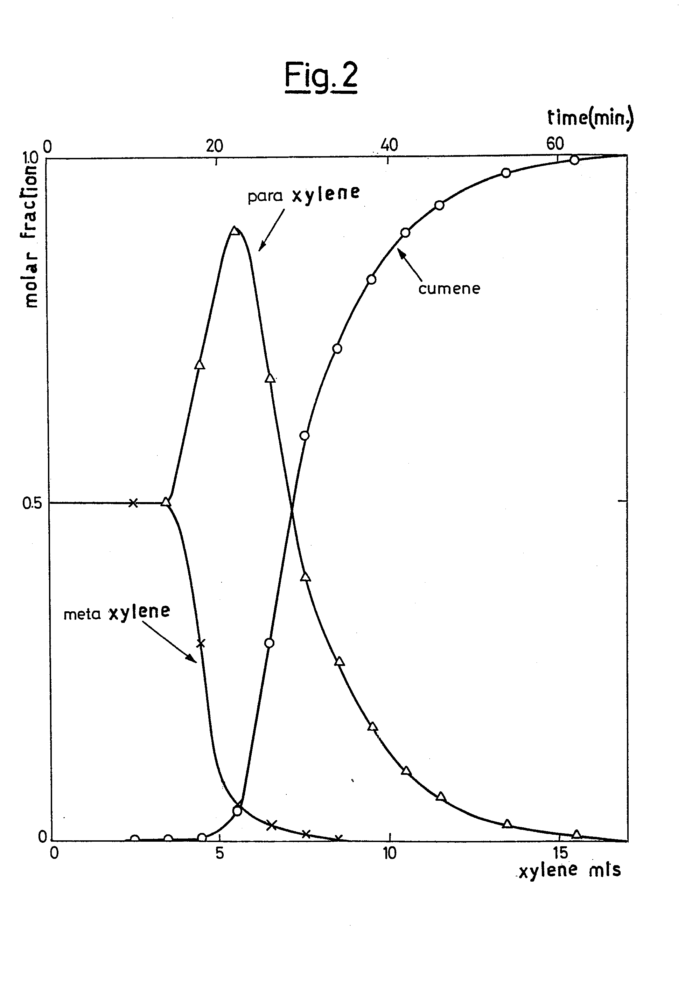

The adsorbent bed having reached the equilibrium, i.e. at the end of the adsorption test, the desorption process was started with isopropylbenzene, always in vapor phase and at the same temperature of 170°C, with the same load of $0.25cm^3$ / min. Metaxylene and paraxylene emerge as seen in Fig. 2.

Even from these curves it is possible to obtain the desorbed amounts of meta and paraxylene that coincide with those as obtained in the adsorption phase. Obviously the selectivity obtained from these tests is confirmed.

The zeolite load capacity results to be equal to about $1.2 \times 10^{-3}$ moles of xylene/g.

Upon examination of the breakthrough curve, obtained in the desorption phase, one can realize that the paraxylene emerges to a concentration which is higher than the one of equilibrium corresponding to 0.5, in molar fraction.

During this phase it takes place an enrichment in paraxilene in the outflowing mixture, with a phenomenon that is typical of the displacement chromatography. The amount of paraxylene being fully separated from metaxylene represents about 10% by weight if compared to the whole desorbed paraxylene.

## EXAMPLE 2

Breakthrough curves either in adsorption and in desorption for a binary mixture of ethylbenzene and paraxylene (1:1 in moles), using isopropylbenzene as desorbent.

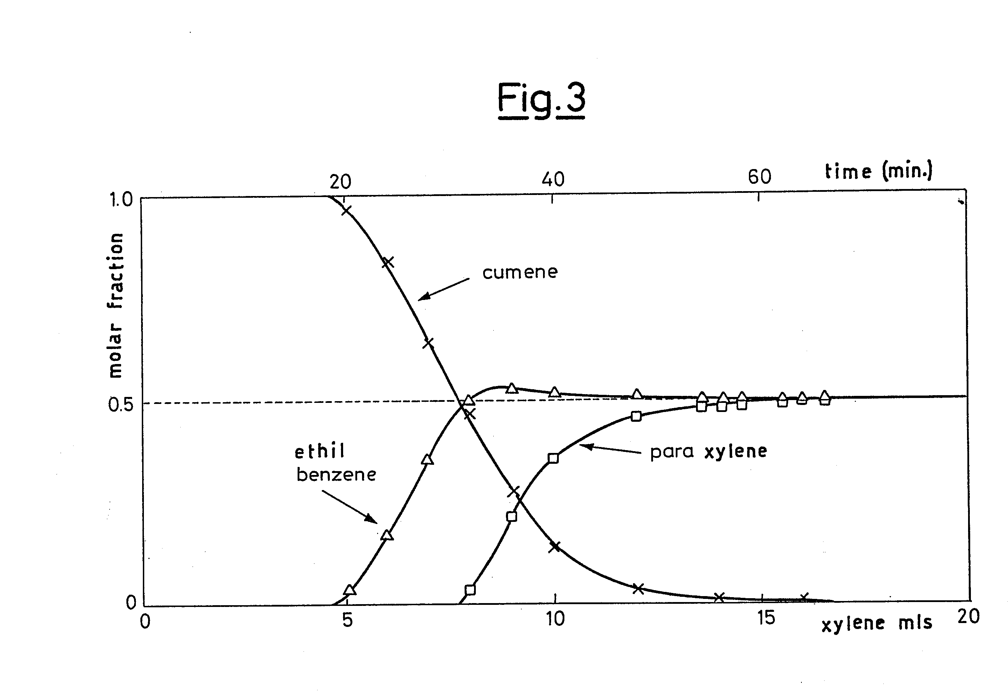

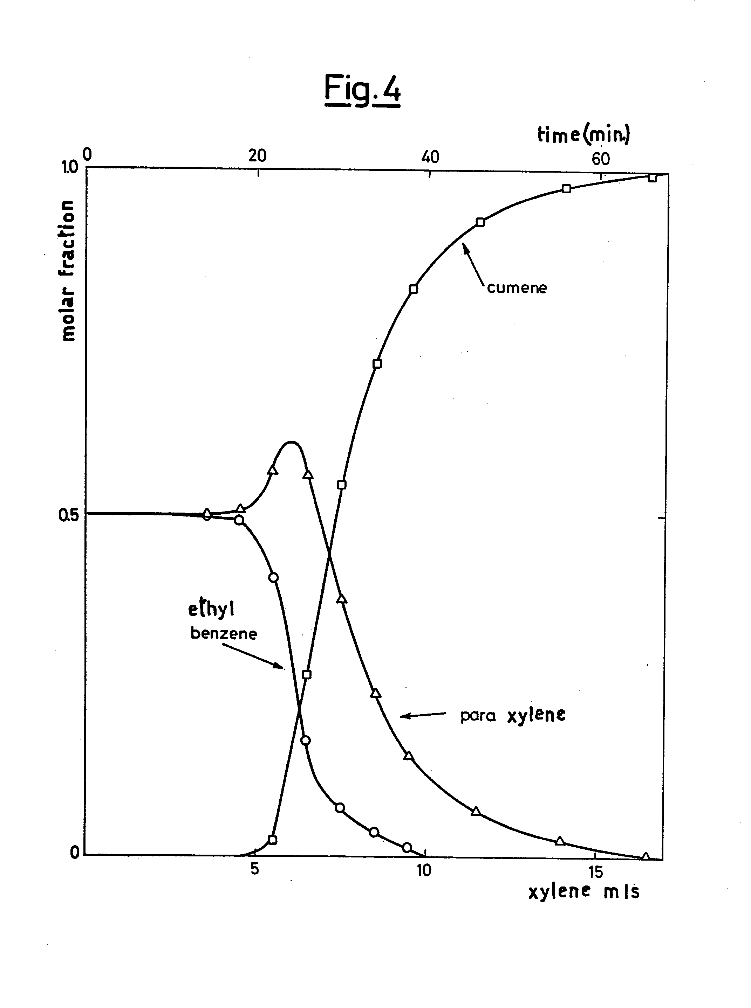

The column is the same as described in the example 1. The operating temperature and the loads are also equal. The breakthrough curves as obtained in the adsorption step are shown in Fig. 3. Those in the adsorption phase with isopropylbenzene are illustrated in Fig. 4. From both graphs it is possible to obtain the amounts of the adsorbed ethylbenzene and paraxylene, said average amount being of $4.6 \times 10^{-4}$ moles/g and $7.2 \times 10^{-4}$ moles/g. The amount of ethylbenzene fully separated from paraxylene in the adsorption phase represents weight about 15% in comparison to the ethylbenzene fed up to that point.

The amount of paraxylene fully separated in the desorption phase represents the 5.5% by weight about of the desorbed total mixure.

Even in this case, as in the previous example, an enrichment of the paraxylene in the desorption phase in comparison to the equilibrium compositions is obtained. The load capacity of zeolite is confirmed.

EXAMPLE 3

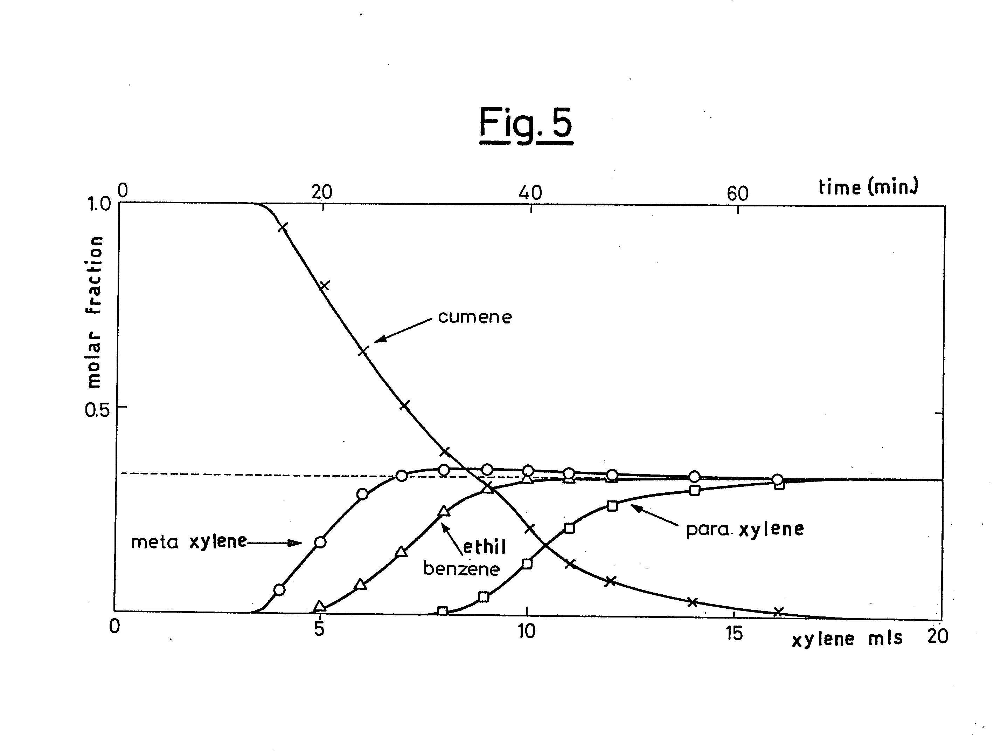

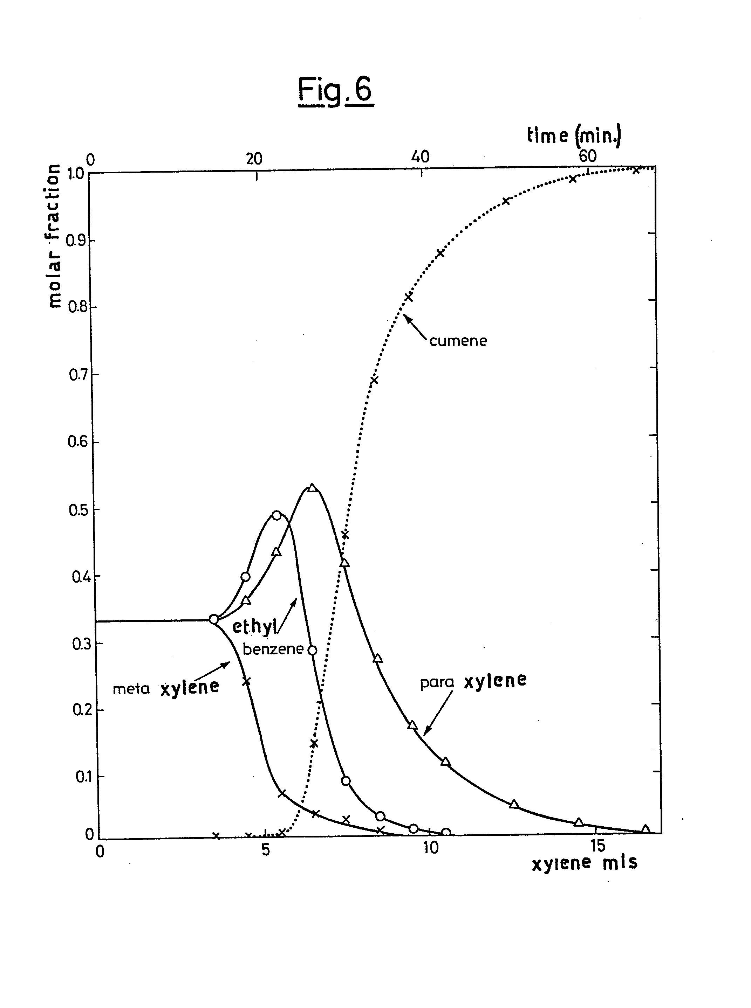

Breakthrough curves either in adsorption and in desorption phase for metaxylene, ethylbenzene and paraxylene ternary mixtures (1:1:1 in moles) using isopropylbenzene as desorbent. The column and the operational conditions are the same in comparison to the previuos examples. The breakthrough curves obtained in the adsorption phase for the three components are shown in Fig. 5, while those obtained for the desorption phase are shown in Fig. 6.

The adsorbed amounts which can be obtained from the two graphs are on an average of $2.3 \times 10^{-4}$ moles/g of metaxylene, $3.6 \times 10^{-4}$ moles/g of ethylbenzene and $5.4 \times 10^{-4}$ moles/g of paraxylene.

Therefore the salectivity data of Table 1 and the zeolite global load capacity are confirmed again.

The metaxylene fully separated in the adsorption phase represents 3.2% of that one fed at that stage.

From the adsorption curves it should be noted how two fractions comprising metaxylene plus desorbent and metaxylene plus ethylbenzene, the compositions of which are reported in Table 2, could be separated.

On the other hand, from the desorption curves it is noticed how it should be possible to recover the 7.5% by weight about of paraxylene in comparison to all of the desorbedparaxylene by means of a composition which is also shown in Table 2.

TABLE 2

Composition of the fractions that can be separated in the breakthrough curves for three components:

| 1st Fraction (adsorption) | metaxylene | ethylbenzene | paraxylene | Desorbent |
|---|---|---|---|---|
| 1st Fraction (adsorption) | 3.7 | | | 96.3 |
| 2nd Fraction (adsorption) | 25.1 | 7.7 | | 67.2 |
| 3rd Fraction (desorption) | | | 3.8 | 96.2. |

If columns having 4m length, of the same diameter and at the same conditions of the example of Fig. 9, the separation shown in Fig. 9 Bis is obtained, from which it is noticeable how it should be possible to separate fractions of the three pure components except for the desorbent.

EXAMPLE 4

Separation of meta and paraxylene binary mixtures (1:1 in moles) by feeding amounts of xylenes being lower than the zeolite load capacity, using the desorbent as fluid carrier, at different loads.

The tests have been carried out always in the same column and at the same temperatures and pressures. On the zeolite bed saturated of isopropylbenzene were sent 4 $cm^3$, measured at 25°C, of the mixture having a ratio 1:1 in moles of meta and paraxylene with a load of 0.206 $cm^3$/min. Upon the xylenes, being fed, the desorbent, isopropylbenzene was passed always with the same load.

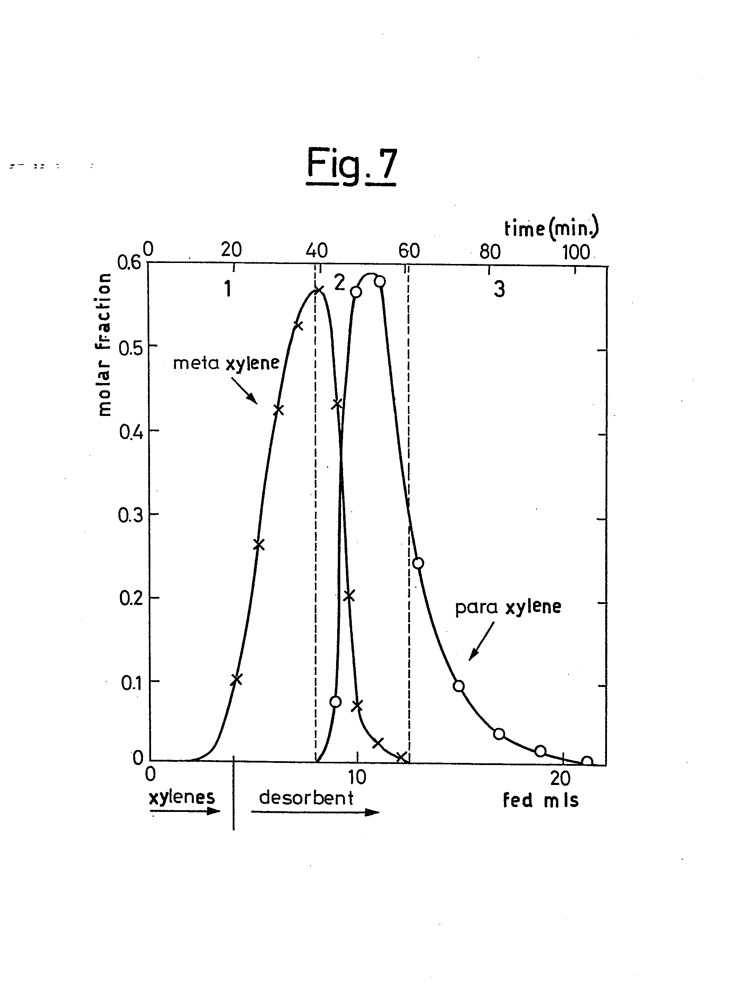

In Fig. 7 it is shown how in the described conditions, meta and paraxylene may flow outside almost completely separated.

A test being fully similar to the previous one, except for the load either of xylene and of desorbent which was kept to 0.5 $cm^3$/min, gave the results that can be seen on Fig. 8.

As it can be noticed, by increasing the linear velocity the duration of the event decreases but the desorbent amount to be used increases and the separation descreases.

By carrying out the separation in this way, three fractions are obtained as per the dashed curves in the figures.

**0093454**

The compositions of these fractions as reported in Table 3, confirm the previous considerations. For a good separation it is suitable, therefore, to operate with spece velocities included between 30 and 600 $h^{-1}$ valves of 100 and 350 $h^{-1}$ being preferred.

TABLE 3

Composition of the fractions being obtained
during the tests shown on Figg. 7 and 8

| Load ($cm^3/m^1$) | Fraction 1 | | | Fraction 2 | | | | Fraction 3 | | |
|---|---|---|---|---|---|---|---|---|---|---|
| | % on total | Distribution | | % on total | Distribution | | | % on total | Distribution | |
| | | meta % | Des.% | | m% | p% | d% | | para% | des.% |
| 0.205 | 30 | 28 | 72 | 23 | 18.4 | 36 | 45.6 | 47 | 7 | 93 |
| 0.5 | 22 | 18.7 | 81.3 | 28 | 19.7 | 24.2 | 56.1 | 50 | 7 | 93 |

- 11 -

0093454

EXAMPLE 5

Separation of ternary mixtures of metaxylene, ethylbenzene and paraxylene (1:1:1 in moles) by feeding xylene amounts lower than the zeolite load capacity and by using the desorbent as fluid carrier.

The column which has been used was the same as in the previous examples, and the temperature and pressure operational conditions remain unchanged.

In a first test 4.5 $cm^3$ of the xylene mixture (metaxylene, paraxylene, ethylbenzene 1:1:1 in moles) have been passed with a load of 0.5 $cm^3$/min. on the as already saturated by isopropylbenzene.

After the feed of xylenes is terminated isopropylbenzene was sent as fluid carrier having the same load. The emerging peaks have the shape that can be seen in Fig 9. It is confirmed that, at the described conditions, meta and paraxylene are almost completely separated.

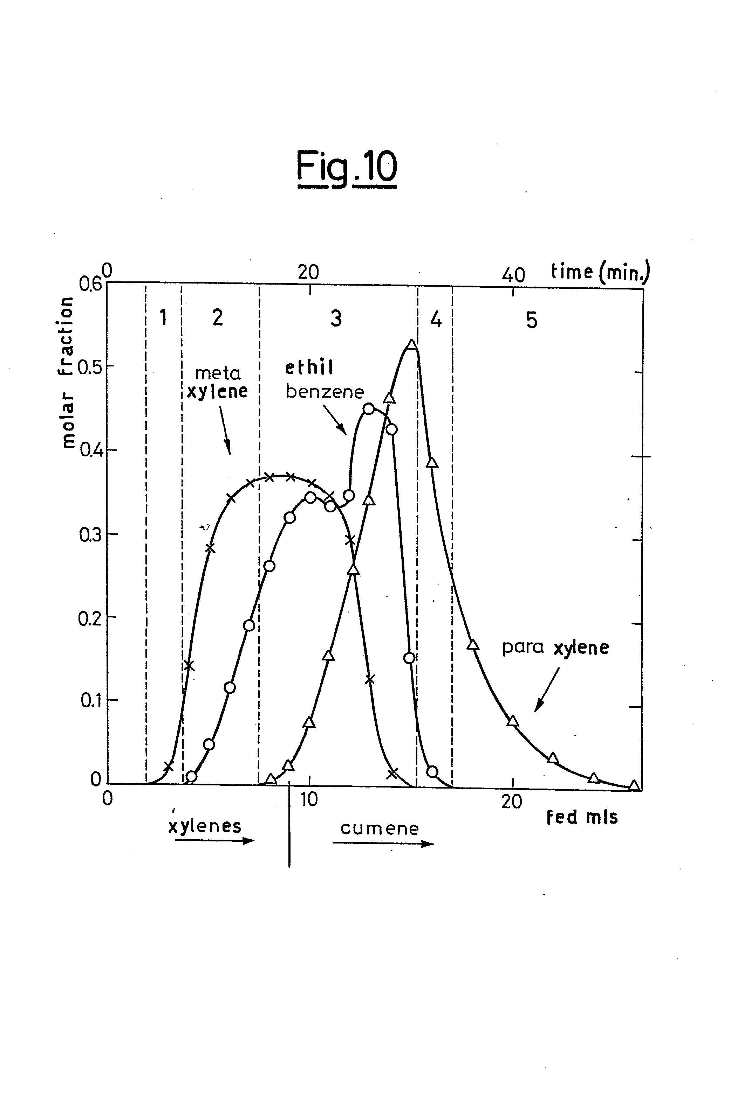

In a further test, under identical conditions, 9 $cm^3$ of xylenes have been fed instead of 4.5 $cm^3$. The results obtained are reported in Fig. 10.

As it can be observed, the separation of the different components is very satisfactory.

From Figures 9 and 10 it is noticeable how it is possible to separate - as it was effectively carried out - five fractions, as shown by dashed lines in the figures having the compositions reported in Table 4. The data of this table confirm the possibility to obtain fractions containing pure metaxylene (fraction 1) and paraxylene (fraction 5), except for the presence of desorbent, together with fractions enriched of the same components (fractions 2.4).

## TABLE 4

Composition of the fractions being obtained
on the tests of Figg. 9 and 10

|  | Fraction 1 | | | Fraction 2 | | | | Fraction 3 | | | | |
|---|---|---|---|---|---|---|---|---|---|---|---|---|
| Fed xylenes | | Distribution | | | Distribution | | | | Distribution | | | |
|  | %on total | m% | des.% | %on total | m% | e% | des.% | %on total | m % | e% | p% | des.% |
| 4.5 | 9 | 2 | 98 | 22 | 23.4 | 7.3 | 69.3 | 19 | 10.1 | 25.4 | 14.8 | 49.7 |
| 9 | 7 | 3.2 | 96.8 | 15 | 29.1 | 9.7 | 61.2 | 32 | 24.3 | 33.3 | 22.4 | 20 |

follows

|  | Fraction 4 | | | | Fraction 5 | | |
|---|---|---|---|---|---|---|---|
| Fed xylenes | | Distribution | | | | Distribution | |
|  | %on total | e % | p % | des. % | %on total | p % | des.% |
| 4.5 | 14 | 1.3 | 20.4 | 78.3 | 36 | 4.7 | 95.3 |
| 9 | 8 | 3 | 35 | 62 | 38 | 6.5 | 93.5 |

m= Metaxylene; p = Paraxylene; e = Ethylbenzene; Des. = Desorbent.

0093454

Just to finalize, the amount of the xylenes to be fed, by following the above mentioned procedure can be encreased up to completely fill the bed, the zeolite load capacity being well known.

## EXAMPLE 6

Comparison between the isopropylbenzene desorbent effect and the one of the other desorbents, especially toluene.

In this purpose a tes has been made for the separation of a binary mixture formed of meta and paraxylene (1:1 in moles) by feeding, as in example of Fig. 8, 4 cm$^3$ of such a mixture on the afore described column and in the same operative conditions of temperature and pressure, with a load of 0.5 cm$^3$/min, the adsorbent bed being saturated of toluene. After feeding of the xylenes is terminated, toluene has been fed as fluid carrier, always with a load of 0.5 cm$^3$/min.

The emerging peaks have the shape as shown on Fig. 11. If one compares this figure with the one concerning the test of Fig. 8 carried out by isopropylbenzene as desorbent, one may conclude that isopropylbenzene is more efficient as desorbent, being lower either the required time to let both components emerge, and the required amounts of the desorbent.

On the contrary the separation does not appreciably decrease, the selectivity remaining in both cases unchanged.

Tests have been made also with other desorbents as for instance benzene and the reaction is quite worse in comparison with toluene; aromatic components having higher molecular weight than cumene have been also tested with good results about the desorbent capacity; however, as they require the temperature is further increased, they cause a lowering in the load capacity of the zeolite and in the separations effect thereof, meant as selectivity.

CLAIMS

**0093454**

1) A process for the separation of aromatic hydrocarbon mixtures in the single components through passage of the mixtures in the vapor phase on an adsorption bed formed of a potassium-exchanged Y zeolite, the latter being previously saturated by desorbent vapors, characterized by the fact that the desorbent is isopropylbenzene, the zeolite material bed being kept at a temperature between 152.5°C and 250°C included, the zeolite bed being treated during the desorption phase with a feed of isopropylbenzene in the vapor phase which separates fractions of the pure components except for the presence of the desorbent and fractions formed of mixtures of two or more of said components in presence of the desorbent.

2) A process according to claim 1, characterized by the fact that said fractions desorbed from the zeolite material bed are mostly enriched in the paraxylene component which quickly separates together with the other components, into cuts wherein the paraxylene molar fraction is prevailingly higher in comparison to that of the other components in the mixture.

3) A process according to claim 1, characterized by the fact that the operating temperature of the zeolite bed is 170°C.

4) A process according to claim 1, characterized by the fact that the operating pressure of the vapors on the zeolite bed is the atmospheric pressure and that the space velocity of the feeding of isopropylbenzene on the same zeolite bed is of between 100 and 350 $h^{-1}$.

5) A process according to claim 1, characterized by the fact that in the adsorption step, wherein such a mixture of the xylenes excepted for orthoxylene is fed on the zeolite bed until the equilibrium is obtained a first fraction containing metaxylene and desorbent and a second fraction containing metaylene, ethylbenzene and desorbent being separated; while in the desorption step, in which the zeolite bed is treated with an equivalent load of isopropylbenzene, a fraction containing paraxylene and desorbent, as well as fractions formed of mixtures of the xylenes and the desorbent prevailingly rich in paraxylene being collected.

6) A process according to claim 1, characterized in that the xylene mixture is fed in lower quantity than the one necessary to saturate the adsorption bed and in that, in the desorption phase with isopropylbenzene, the single pure components, excepted for the desorbent, and their mixtures prevailingly richer in paraxylene, in presence of desorbent are collected.

7) A process according to claim 6, characterized in that a metaxylene, ethylbenzene and paraxylene ternary mixture is fed in lower quantity that the one necessary to saturate the zeolite bed, and in that in the desorption phase five fractions are obtained, the first and the last of which containing metaxylene and desorbent and respectively metaxylene and desorbent, the three intermediate fractions being formed of xylene mixtures in presence of desorbent.

8) A process according to claim 7, characterized by the fact that fractions of the three pure components, except for the desorbent, are further separated.

Fig.1

# Fig.2

Fig.3

# Fig.4

# Fig.5

0093454

0093454

# Fig.6

# Fig. 7

8/12

0093454

# Fig.8

Fig.9

# Fig. 9 bis

0093454

# <u>Fig.10</u>

Fig.11

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,Y | EP-A-0 045 953 (SISAS)<br>* Claims *<br>--- | 1-8 | C 07 C 7/13<br>C 07 C 15/08 |
| Y | CHEMICAL ABSTRACTS, vol. 92, no. 21, 26th May 1980, page 616, no. 180802u, Columbus, Ohio, USA<br>& JP - A - 79 38089 (TORAY INDUS-TRIES, INC.) 19-11-1979 * Abstract *<br>----- | 1 | |

|  |  |
|---|---|
|  | TECHNICAL FIELDS SEARCHED (Int. Cl. ³)<br><br>C 07 C 7/00 |

The present search report has been drawn up for all claims

| Place of search | Date of completion of the search | Examiner |
|---|---|---|
| THE HAGUE | 03-08-1983 | VAN GEYT J.J.A. |